# EUROPEAN PATENT SPECIFICATION

(11) **EP 4 395 708 B1**
(45) Date of publication and mention of the grant of the patent: **12.08.2026**
(21) Application number: 22765246.8
(22) Date of filing: 01.09.2022
(51) Int. Cl.: A61F 2/16, A61F 9/007

(54) **INTRAOCULAR LENS REMOVER AND METHODS OF MANUFACTURING IT**
VORRICHTUNG ZUR ENTFERNUNG VON INTRAOKULARLINSEN UND VERFAHREN ZUR DEREN HERSTELL£UNG
DISPOSITIF DE RETRAIT DE LENTILLE INTRAOCULAIRE ET METHODE POUR LE FABRIQUER

(30) Priority: 01.09.2021 NL 2029101
(43) Date of publication of application: 10.07.2024
(73) Proprietor: Ophtec B.V., 9728 NR Groningen (NL)
(72) Inventor: SIMON, Harry Franciscus, 9728 NR Groningen (NL); ZIJLSTRA, Tjalke Jaddai, 9728 NR Groningen (NL); SPEELMAN, Roelof, 9728 NR Groningen (NL); DUSSELJEE, Jan Hendrik, 9728 NR Groningen (NL); IDEMA, Floris Wigbolt, 9728 NR Groningen (NL)
(74) Representative: Arnold & Siedsma
(86) International application number: PCT/NL2022/050496
(87) International publication number: WO 2023/033648

(56) References cited:
- EP-A2- 1 820 474
- WO-A1-2015/105465
- US-A1- 2003 088 253
- US-A1- 2014 135 820
- US-A1- 2014 358 155

## Description

The invention relates to an intraocular lens remover whereby an intraocular lens, or IOL, can be removed from an eye. Such a lens is usually inserted into the eye via an incision and secured in situ using haptic elements. Such lenses are also referred to as implant lens or artificial lens, and are implanted to replace the original lens or optionally as addition thereto.

It may be necessary or desirable for various reasons to remove a previously placed intraocular lens from the eye. For this purpose an incision is made in the eye to enable removal of the lens. In order to keep the dimensions of the incision as small as possible lens is for instance folded and/or cut in the eye in order to reduce the dimensions of the lens before it is passed through the incision out of the eye. A number of operations are required for this, these requiring great precision in order to avoid damaging the eye when removing the lens. In order to reduce this risk use is made of auxiliary tools in the removal of the intraocular lens. Dr. Fukuoka (Handaya Co.) has for instance developed a tool provided with a gripper element which engages on the lens and whereby the lens can then be passed through the incision. Another tool is described in WO 2020/150213 A1 and relates to a guide through which a separate gripper can be passed in order to engage on the lens. After manipulation with the gripper and retracting of the gripper in the guide, the whole is passed back through the incision in order to remove the lens from the eye. A drawback of such a tool is that at least a part of the relevant movements with the lens for removing must be performed in the immediate vicinity of the endothelium, which entails a risk of inflicting irreparable damage thereto.

US 2003/0088253 A1 discloses a dual action ophthalmic implant extraction. Also, US 2014/0135820 A1, EP 1 820 474 A2 and WO 2015/105465 disclose extractors.

The present invention has for its object to obviate or at least reduce the above stated problems and to provide an intraocular lens remover with associated method which is further preferably simple and safe in use.

This object is achieved with an intraocular lens remover according to claim 1.

The lens remover according to the invention is provided with a holder whereby a user can hold the lens remover and wherein a gripper is movably provided. The gripper is provided at the outer end with a gripper element configured to engage on the lens for removing. This engagement can be realized in various ways, for instance with a hooked element, a clamping element and/or another suitable element. If desired, the lens is positioned relative to the gripper element using an auxiliary tool, such that the gripper element engages on the lens for removing. The gripper is provided movably relative to the holder and can be operated with a gripper operating element.

After the lens remover has been inserted through the incision, the gripper can be moved relative to the holder such that the gripper element exits the holder and can engage on the lens. After the lens has been gripped, the gripper can be moved back again relative to the holder such that the lens is moved inside and the lens remover with lens can be passed through the incision.

According to the invention, an (actively) moveable cartridge is further provided which is moveable relative to the holder and the gripper, particularly moveable relative to the gripper element. The cartridge is here provided to be moved wholly or at least partially over the gripper element, and particularly the lens brought into contact therewith, by means of a relative translating and/or rotating movement. By moving the cartridge relative to the holder and the gripper element, preferably once the gripper element is in contact with the lens for removing, the folding and/or stretching of the lens as a result of the movement of the cartridge can be performed at a safe distance from the endothelium in particular, thus reducing or even wholly obviating the risk of damage thereto. The folding and/or stretching of the lens can additionally be performed in controlled manner without the holder for instance having to be moved. This movement of the cartridge is performed with the cartridge operating element, such that a user is able to move the cartridge in effective manner. In currently preferred embodiments the lens is moved inside while and/or after a folding movement is performed, wherein the lens is preferably stretched in a longitudinal direction of the remover. This preferably makes the lens smaller. A compact remover, and therefore an incision of minimal size, can hereby suffice.

Said operating elements can be provided on or at the remover at different positions, for instance centrally on the holder or at a different position on the remover, in order to thus realize the best possible convenience of use for a user.

According to the invention, the cartridge is provided movably relative to both the holder and the gripper, and particularly relative to the gripper element. This enables an effective and safe movement between these components of the lens remover, wherein the risk of damage to the eye is limited further. The cartridge is here preferably configured to fold the lens. This folding is realized during the relative movement of the cartridge over the gripper element with the lens, wherein the lens is preferably also stretched to some extent. The intraocular lens remover is configured to enable the folding (and optional stretching) to be performed in the (human) eye. The lens is here preferably folded in the (human) eye, at or in the outer end of the cartridge. The result of this folding and optional stretching movement of the lens is that the dimensions of the lens are reduced and an incision of limited size can suffice. Owing to the high degree of control over said relative movements, the dimensions of the lens can likewise be reduced in controlled manner within a fairly limited range. This makes it possible to reduced the incision further still, particularly to an incision with a length of less than 3.5 mm, preferably even less than 3.2 mm.

In an advantageous embodiment according to the invention the lens remover comprises a cartridge translation mechanism configured to translate the cartridge relative to the holder and gripper.

Using the cartridge translation mechanism, the cartridge can slide relative to the holder and the gripper in effective manner, and here particularly slide over at least a part of the gripper element and thereby over a lens. This cartridge translation mechanism is preferably actively operated using the cartridge operating element. This cartridge operating element is optionally provided integrally with the cartridge. This cartridge operating element is alternatively arranged on or at the holder via a separate mechanism.

In a further advantageous embodiment according to the invention the lens remover comprises a cartridge rotation mechanism configured to rotate the cartridge relative to the holder and gripper.

By providing a cartridge rotation mechanism the cartridge can rotate to some extent relative to the gripper and particularly the gripper element. Tests have shown that this makes it simpler to move the cartridge over the gripper element with the lens, also if the lens is not positioned optimally relative to the gripper element and may give a greater resistance when being introduced into the cartridge. It has particularly been found that rotating the cartridge while it moves over the gripper element and the lens considerably reduces the resistance of the lens to the deformation while being introduced into the cartridge. Not only does this increase the convenience of use, it also reduces the risk of damage to the eye.

The cartridge rotation mechanism is optionally integrated with the cartridge itself and can be activated by direct engagement of the user on the cartridge. Alternatively, the cartridge is rotatable using a mechanism and an operating element arranged on or at the holder.

In a further advantageous embodiment according to the invention the remover is provided with a cartridge guide which is connected operatively to the cartridge and is configured to guide the cartridge in a combined translating and rotating movement.

By simultaneously performing a translating and rotating movement of the cartridge relative to particularly the gripper element a controlled movement of the cartridge over the lens is enabled, wherein the risk of additional resistance of the lens to the desired deformation thereof when being introduced into the interior of the cartridge is reduced further. Providing a cartridge guide intended for this purpose can further increase the convenience of use for a user. The cartridge preferably makes use of a helical or twisting movement relative to the holder and the gripper, particularly the gripper element. It has been found that such a movement makes it possible to introduce the lens into the interior of the cartridge in a deformed state in effective manner in the eye. In one of the currently preferred embodiments according to the invention the operation of the cartridge translation mechanism and the cartridge rotation mechanism is integrated in the cartridge operating element. This further increases the convenience of use.

In an advantageous embodiment according to the invention the lens remover comprises an integrated operating element in which the gripper operating element and the cartridge operating element are integrated.

Integrating the operating elements of the gripper and the cartridge in a combined operating element further increases the convenience of use for the user. Additionally, the operation of the remover is simplified further so that operation thereof is unambiguous. A further advantage of providing an integrated operating element is that a user can hold the remover in the same way during the whole process and need for instance not take hold of the holder in a different position to be able to operate elements of the remover. This increases the general level of control in removal of a lens from the eye.

In a currently preferred embodiment the integrated operating element is provided with a guide track for movement of the cartridge and an additional guide for movement of the gripper. By providing a guide track for the cartridge and a separate guide for the gripper the desired movement of the individual elements can thereby be adapted. It has been found that it is mainly provision of a guide track that enables a controlled movement of the cartridge in particular. It is possible here to combine the translating movement of the cartridge with a rotating movement of the cartridge in order to further facilitate the removal of a lens.

In a further advantageous embodiment according to the invention the lens remover comprises a holder rotation element configured to engage on the holder for rotating the holder relative to the cartridge thereby.

Providing a holder rotation element enables the cartridge and the holder to be rotated relative to each other in effective manner. Providing a separate holder rotation element enables a user to rotate the cartridge relative to the holder as desired, for instance depending on the actual positioning of the lens on the gripper element. This increases the flexibility for the user during removal of a lens.

In a further embodiment according to the invention the lens remover comprises one or more lens guides configured to wholly or partially fold the lens with the gripper element.

Providing the lens guides which are preferably operatively connected to the gripper element enables the lens for removing to be positioned relative to the gripper element and the cartridge in effective manner, after which the cartridge can be moved thereover in effective manner using a translating and/or a rotating movement. The lens guides here preferably provide for the lens for removing being placed and held in position and, together with the gripper element, make it possible to wholly or partially fold and possibly stretch the lens and optionally pre-fold it prior to the lens being introduced into the interior of the cartridge.

One of the one or more lens guides is preferably provided with a pressing form. Such a pressing form makes it possible to press the lens into the pressing form to some extent and preform it in the desired direction, preferably with a bend directed away from the endothelium, and particularly to pre-fold it before it is introduced into the interior of the cartridge. Such a pressing form is for instance a cup shape. Such a cup shape is optionally provided with an opening in which the lens can be partially arranged in order to thereby fix the lens in position. Experiments have shown that a pressing form, and particularly a cup shape, ensures in effective manner that the lens is pre-folded and optionally already stretched to some extent, so it can then be introduced into the interior of the cartridge by the movement of the cartridge relative to the gripper element. In a possible embodiment the gripper element is provided here with a forming element in order to achieve this pre-folding of the lens during positioning of the lens in the cartridge. A controlled deformation of the lens is here simplified further and becomes better controllable.

In a further advantageous embodiment according to the invention the gripper element is configured to engage on the lens in a substantially upward direction.

Having the gripper elements engage on the lens in a substantially upward direction further reduces the risk of damage to internal eye (parts) during use of the lens remover. Because the operating element is preferably also provided on the upper side, or the side directed substantially upward in use, of the holder, the gripper element moves substantially in this upward direction.

In a further advantageous embodiment according to the invention the gripper element is arranged at an angle to the holder.

Arranging the gripper element at an angle to the holder enables adjustment of the position of the holder relative to the eye during removal of the lens from the eye. Providing such an angle thus for instance makes it easier to work on so-called deep-set eyes with the lens remover when removing a lens. If desired, the angle can be adjusted to the actual requirement and shape.

In a further advantageous embodiment according to the invention the lens remover comprises a film configured to surround the lens and a film guide mechanism configured to move the film relative to the gripper.

Providing a film makes it possible to at least partially surround the lens with said film after it is engaged with the gripper. Surrounding here also comprises enclosing and enveloping. This realizes a shield between the lens and internal parts of the eye. This reduces the risk of damage to such parts. In a currently preferred embodiment the cartridge can move over the film arranged over the lens, wherein the lens is folded into the interior of the cartridge and is preferably also stretched.

The invention also relates to a method for manufacturing an intraocular lens remover according to claim 1.

It will be apparent that such manufacture can be realized in diverse ways, wherein use is for instance made of so-called injection moulding and compression moulding and 3D printing.

Further advantages, features and details of the invention are elucidated on the basis of preferred embodiments thereof, wherein reference is made to the accompanying drawings, in which:
- Figure 1A is a side view of a lens remover in an embodiment according to the invention;
- Figure 1B is a detail view of the gripper element of the lens remover of figure 1A;
- Figure 1C is a view of a lens remover in an alternative embodiment according to the invention;
- Figure 1D is a view of a lens remover in an alternative embodiment according to the invention;
- Figure 2A is a further embodiment of a lens remover according to the invention;
- Figure 2B is a view of an alternative embodiment with alternative operating element for the lens remover of figure 2A with the gripper element shown therein;
- Figure 2C is a section of a further alternative lens remover;
- Figure 3A is a view of yet another embodiment of the lens remover according to the invention;
- Figure 3B is a section of the lens remover of figure 3A;
- Figure 3C is a view of the lens remover of figures 3A-B, further provided with a cartridge;
- Figure 4 is a view of an alternative embodiment of the lens remover according to the invention;
- Figures 5A-C are views of a further alternative embodiment of a lens remover according to the invention;
- Figures 6A-D are views of a further alternative embodiment of a lens remover according to the invention; and
- Figures 7 and 8 are views of alternative drive mechanisms for a lens remover according to the invention.

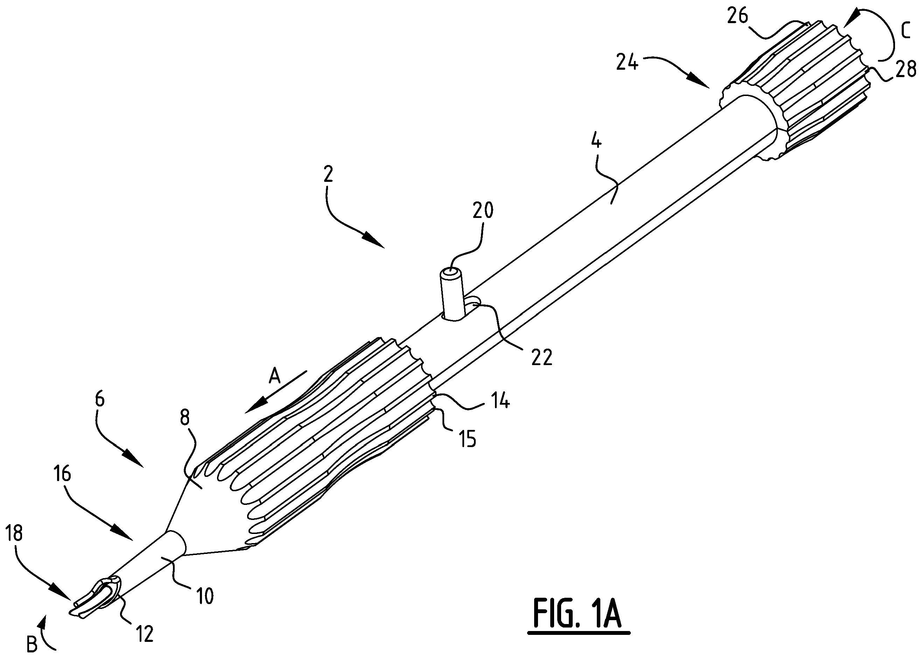

Lens remover 2 (figure 1A) is provided with holder 4. Provided at first outer end 6 of holder 4 is cartridge 8, which can translate in direction A and can rotate in direction B relative to holder 4. Cartridge 8 is provided with outer end 10 which, in use, is positioned at the incision in the eye. In the shown embodiment outer end 10 is provided with deforming element 12 whereby a lens is folded and optionally stretched when cartridge 8 moves over this lens. In the shown embodiment cartridge 8 is further provided with cartridge operating element 14 in the form of arranged ribs 15 for giving the user sufficient grip on cartridge 8.

Further arranged in holder 4 is gripper 16 with gripper element 18 arranged on front side 6. Operating element 20 is arranged in recess 22 in holder 4 and can be moved between a retracted (rear) position, wherein gripper element 18 is positioned at least partially in cartridge 8 and/or holder 4, and an active (front) position (shown in figure 1A) in which gripper element 18 protrudes further from outer end 10 of cartridge 8. In the shown embodiment holder 4 is further provided on rear side 24 with holder rotation element 26, preferably provided with grip elements 28. Holder rotation element 26 can rotate in direction C and makes it possible to realize a relative movement between gripper element 18 and cartridge 8.

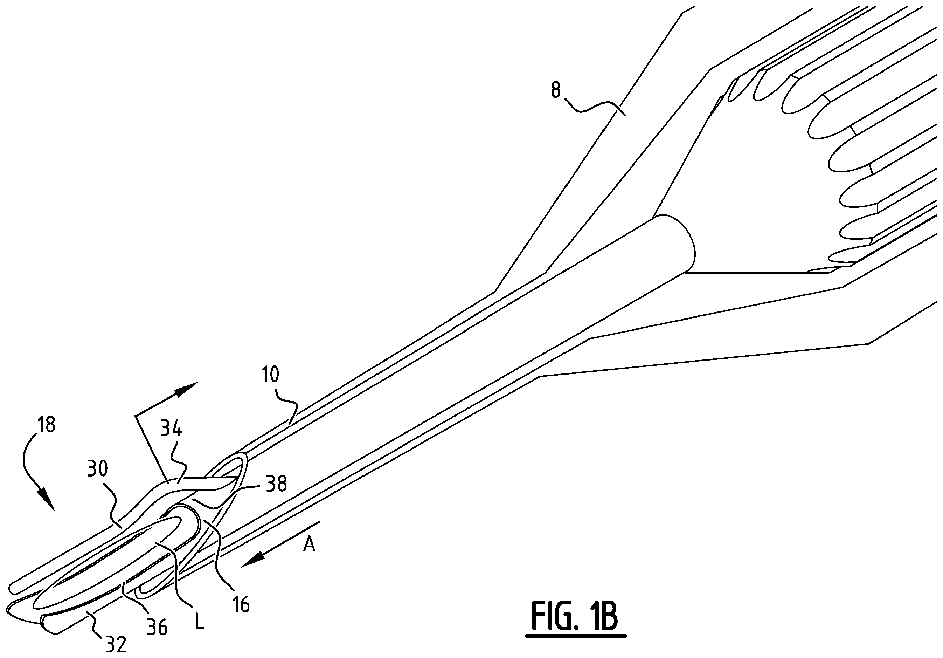

Gripper element 18 (figure 1B) is provided with gripper 30 and longitudinal guide/lens guide 32. Schematically shown is lens L, which is already positioned in gripper element 18, optionally making use here of an additional auxiliary tool. Gripper 30 is further provided with forming element 34 in the form of a curve. When cartridge 8 is slid over gripper element 18 and lens L in direction A, forming element 34 is pressed downward and thereby imparts an (additional) pressing effect on lens L into the cup shape 36 of longitudinal guide(s) 32. **In** order to enable this movement groove 38 is provided in gripper element 18. It will be apparent that groove 38 can take different forms, including a spiral groove.

When removing lens L using lens remover 2, and incision must first be made in the eye. Lens remover 2 is then placed relative to the incision, wherein outlet 10 is positioned at the incision such that frontside 12 extends into the eye. Following this positioning, handle 20 is slid forward (as shown in figure 1A), whereby gripper 30 of gripper element 18 is likewise moved forward and protrudes on the front side of lens remover 2. Lens L is then positioned in gripper element 18 (figure 1B). After lens L is positioned in gripper element 18, cartridge 8 is moved forward in direction A by the user by engaging on elements 14, 15, whereby outlet 10 is moved further through the incision, wholly or partially over gripper element 18 with lens L, while holder 4 remains in the same position. Gripper 30 is here pressed further against lens L in that form 34 is pressed toward lens L when cartridge 8, particularly outlet 10 thereof, is slid over gripper element 18. Lens L is hereby preformed, particularly pre-folded, to some extent. Cartridge 8 is further slid on in direction A such that gripper element 18 is introduced largely or wholly into the interior of cartridge 8. Holder 4 is here preferably fixed in this same position. This makes it possible also to hold lens L at the same position in the eye while cartridge 8 is slid thereover. When placing cartridge 8 over lens L, the operator can further also rotate this cartridge 8 in direction B in order to reduce the resistance to deformation of lens L. Alternatively or additionally, a user can also use holder rotation element 26 for the relative (rotation) movement of holder 4 and gripper element 18. Handle 20 can be moved back prior to or during movement of cartridge 8 in order to retract gripper element 18 further. After handle 20 is retracted and cartridge 8 moves forward with an optional rotating movement, holder 4 of remover 2 can be retracted out of the incision, wherein the lens is co-displaced into the interior. In the shown embodiment handle 20 is preferably moved back prior to the movement of cartridge 8 in order to position lens L relative to guides 32.

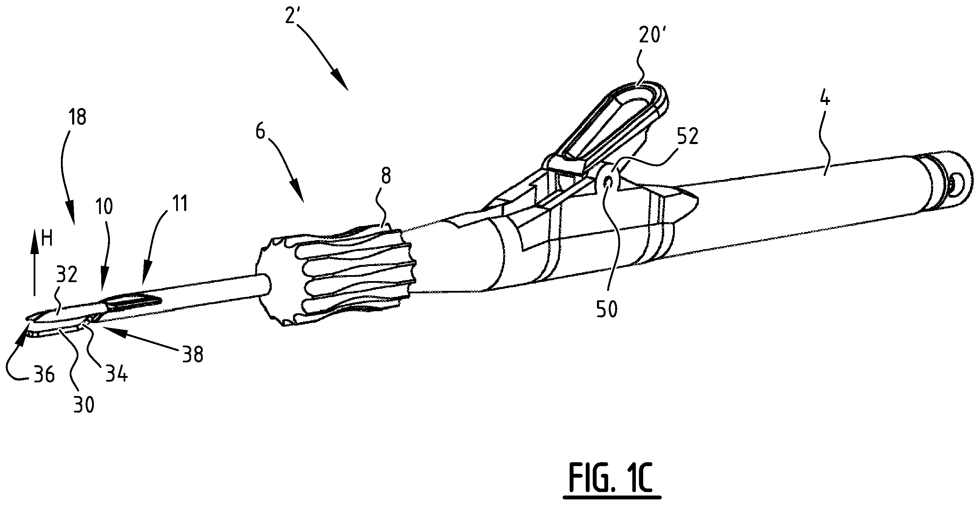

In an alternative embodiment lens remover 2' (figure 1C) is provided with holder 4 and cartridge 8 at first outer end 6 of holder 4. A difference with lens remover 2 (figures 1A-B) is formed by the absence of handle 20, which has been replaced by operating element 20'. A further difference is formed by the orientation of gripper element 18 which is provided with gripper 30 and longitudinal guide 32, which is reversed relative to the orientation of lens remover 2 (as compared to figure 1B). Handle 20' is configured to pull in gripper 30. Gripper 30 is further provided with forming element 34 in the form of a curve. After a lens has been gripped by gripper 30, forming element 34 is pressed upward in direction H when cartridge 8 slides over gripper element 18 and thereby imparts an (additional) pressing effect to a lens present in cup shape 36 of longitudinal guide(s) 32. In order to enable this movement groove 38 is provided in gripper element 18. As stated above, a different orientation is provided in this shown embodiment, wherein gripper 30 is rotated relative to the gripper in lens remover 2 (figures 1A-B) through about 180 degrees. This has the advantage that, when removing a lens, the risk of damage to (parts of) the eye are minimized. It will be apparent to the skilled person that this orientation can also be applied to other embodiments of the lens remover.

Operating element 20' (figure 1C) is rotatable round shaft 50 which is arranged in and/or between shaft holders 52 of holder 4. Pressing element 20' (figure 1C) causes gripper 30 to make its way at least partially into cartridge 8. Gripper 30 is here pressed further against the lens in that form 34 is pressed further against the lens in the relative movement of gripper 30 relative to cartridge 8, particularly outlet 10 thereof, which is provided with recess 11 and/or groove 38. The lens is hereby preformed, particularly pre-folded, to some extent. Cartridge 8 is further slid on such that gripper element 18 becomes positioned largely or wholly in the interior of cartridge 8. This operation corresponds for the most part with the operation of lens remover 2 (figures 1A-B). For realizing the movement of gripper 30 element 20' (figure 1C) is operatively connected with an outer end to a translation mechanism. This enables the translating movement of gripper 30. A rotating movement of gripper 30 is preferably realized simultaneously to this translating movement by a rotation mechanism, for instance embodied using a pin-groove mechanism. With the rotation mechanism the translating movement of gripper 30 is combined with the rotating movement of gripper 30 in effective manner. It will be apparent that the translation mechanism and rotation mechanism can be embodied in different ways. Examples of such mechanisms are shown in further embodiments.

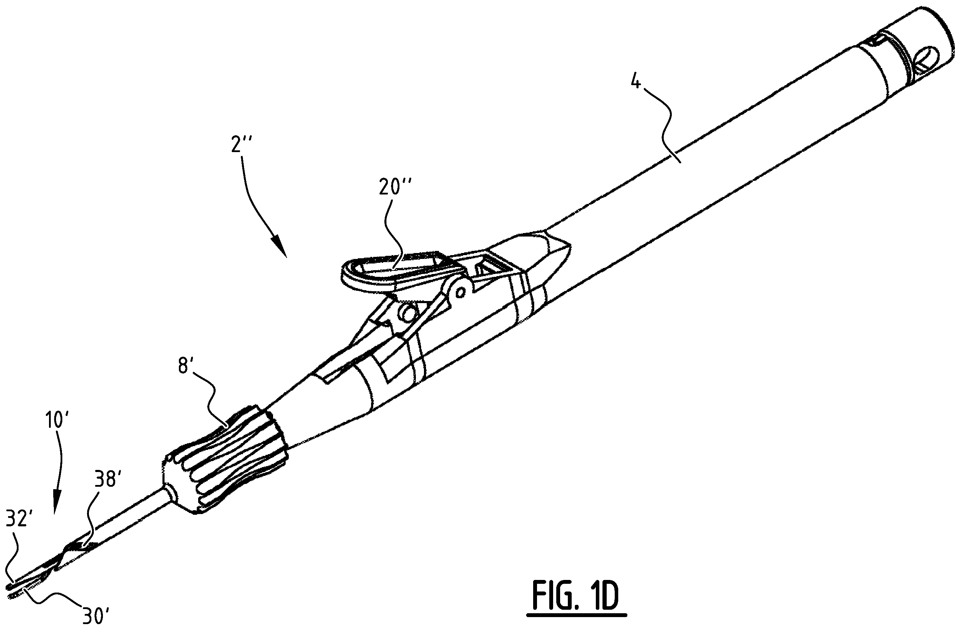

Lens remover 2' (figure 1C) shows handle 20' whereby gripper 30 and longitudinal guide(s) 32 are moved toward each other. In alternative lens remover 2" (figure 1D) said elements are likewise moved toward each other by pressing handle 20". This movement can be realized by for instance moving guides 32, 32' forward by pressing handle 20', 20", and/or by retracting gripper 30, 30' back in the direction of holding 4 by pressing handle 20', 20". It will be apparent that diverse options can be realized in the different embodiments. Spiral groove 38' is further provided in lens remover 2" (figure 1D). Spiral groove 38' can be arranged twisting to the left or to the right and be adapted to an intended user. It will be apparent that the diverse embodiments for such a groove shown in the different embodiments are interchangeable and/or combinable.

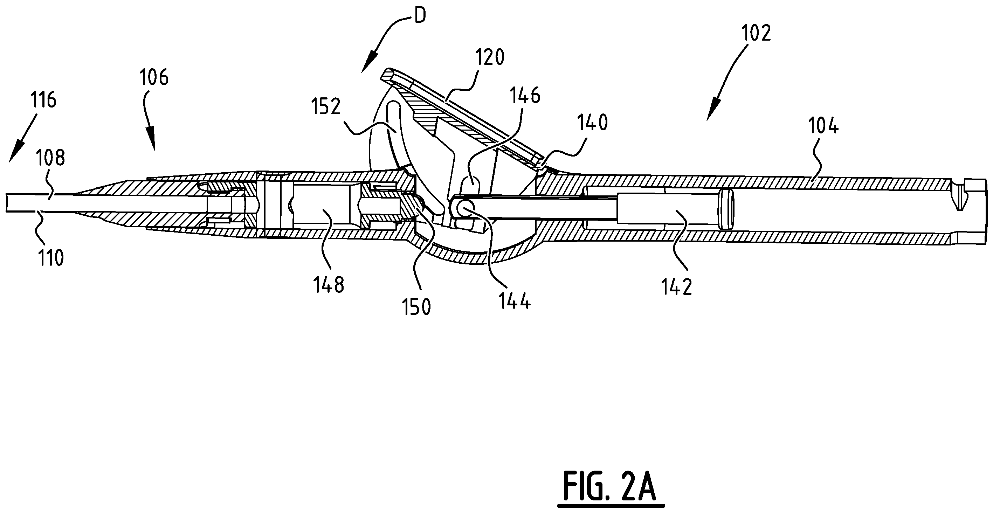

In an alternative embodiment of lens remover 102 (figure 2A) holder 104 is provided, with arranged on outer end 106 thereof cartridge 108 with outer end 110. In this shown embodiment operating elements for cartridge 108 and gripper element 118 of gripper 116 are integrated in operating element 120. Movements of gripper element 118 and cartridge 108 are thereby also integrated. Use is made here of gripper mechanism 142 which is operatively connected via connection 144 in groove or guide track 146 of operating element 120. Operating element 120 is connected pivotally to holder 104 with hinge 140. Cartridge mechanism 148 moves cartridge 108 relative to holder 104, wherein mechanism 148 is arranged in guide track 152 of operating element 120 using coupling or connection 150. By pressing element 120 in direction D mechanism 148 with cartridge 108 moves forward in the direction of outer end 106, wherein gripper element 118 is also moved using mechanism 142.

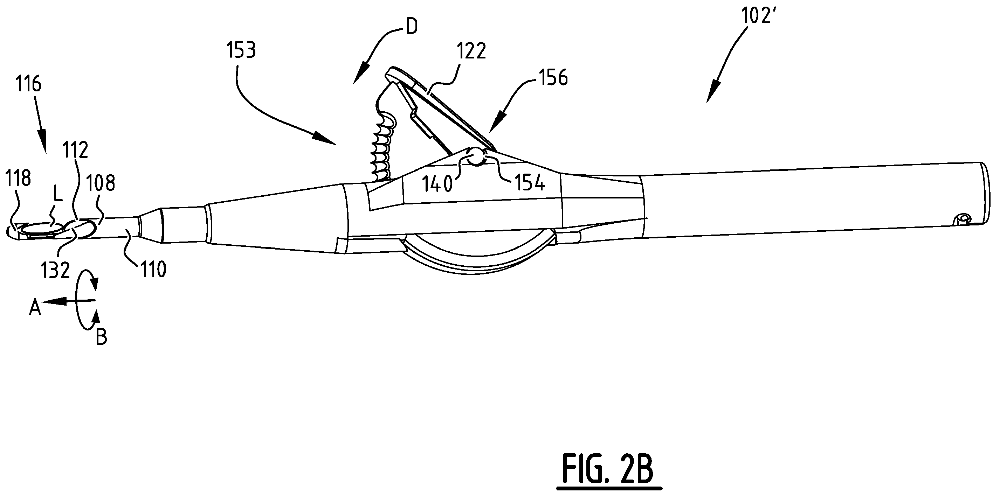

Alternative embodiment 102' (figure 2B) has the same type of action, wherein a partial toothed wheel 153 is provided for the movement of cartridge 108. By moving operating element 122 in direction D a translating and/or rotating movement of cartridge 108 is brought about using toothed wheel 153 and associated toothed wheel (part) on cartridge mechanism 148 (not shown).

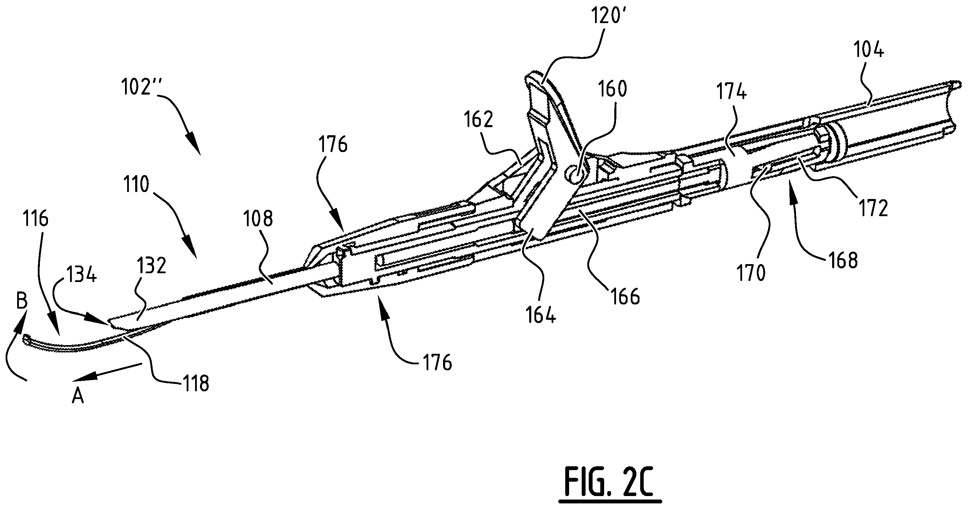

A further alternative embodiment 102" (figure 2C) is provided with operating element 120' rotatable round shaft 160 which is arranged in and/or between shaft holders 162 of holder 104. Pressing element 120' (figure 2C) moves cartridge 108 at least partially over gripper 116. Gripper 116 is here pressed further against the lens in that gripper element 118 pushes the lens into or against cup shape 134 when cartridge 108, particularly outlet 110 thereof, moves over gripper element 118. The lens is hereby preformed, particularly pre-folded, to some extent. Cartridge 108 is further slid along such that gripper element 116 is introduced largely or wholly into the interior of cartridge 108. In order to realize the movement of gripper 116 element 120' is operatively connected with outer end 164 to translation mechanism 166, which is operatively connected to gripper element 118. This enables the translating movement of gripper 116. A rotating movement of cartridge 108 is preferably realized by rotation mechanism 168 simultaneously to this translating movement, wherein pin 170 moves in keyway 172 in rotation element 174 as a kind of pin-groove mechanism. Rotation mechanism 168 is connected operatively to cartridge 108. A pin-groove or screw thread connection 176 is preferably provided on the front side of rotation mechanism 168 for guiding the rotating movement. With rotation mechanism 168 the translating movement of cartridge 108 relative to gripper 116 is combined with the rotating movement of cartridge 108 in effective manner by translation mechanism 166.

Lens removers 102, 102', 102" are provided with respective operating elements 120, 120', 122 which, in the shown embodiment, are pivotally connected to this holder 104 using pin 140 arranged in the recess 154 in holder 104. In the shown embodiment grippers 116 and gripper elements 118 are embodied in the same way for lens removers 102, 102', 102". For this purpose lens L (figure 2B) is placeable in gripper 116 and is here held by gripper element 118. Gripper 116 is here provided with guides 132. Cartridge 108 is provided with folding element 112.

In use of lens removers 102, 102', 102" an incision is first made in the eye. Removers 102, 102', 102" are then placed (partially) in the incision, wherein cartridge outlet 110 extends (partially) through the incision and is here therefore located at the position of this incision. When inserting remover 102, 102', 102", handle 120, 122 is preferably pressed, for instance in direction D, so that gripper 116 lies wholly or partially in the interior of remover 102, 102', 102". After positioning of remover 102, 102' with outer end 106 in the eye, operating element 120, 122 is in this embodiment released. Preferably provided is an optional spring element (shown schematically with element 156) whereby element 120, 122 moves back again. It is noted here that such an optional spring element 156 is also applicable in the other embodiments. Lens L can then be manipulated and introduced into gripper element 118, optionally with an external auxiliary tool. By then for instance once again pressing operating element 120, 122 in direction D, here for instance counter to an optional spring action, gripper element 118 is pulled inward slightly, whereby the lens is pre-folded using guides 132, and cartridge 108 is preferably moved forward in controlled manner with a translating and preferably likewise slightly rotating movement, wherein forming element 112 rotates through a small angle and folds lens L further and preferably stretches it to some extent so as to introduce it into the interior of cartridge 108. During this movement holder 104 remains in the same position. Following this, lens remover 102, 102', 102" is retracted from the incision and lens L is removed from the eye.

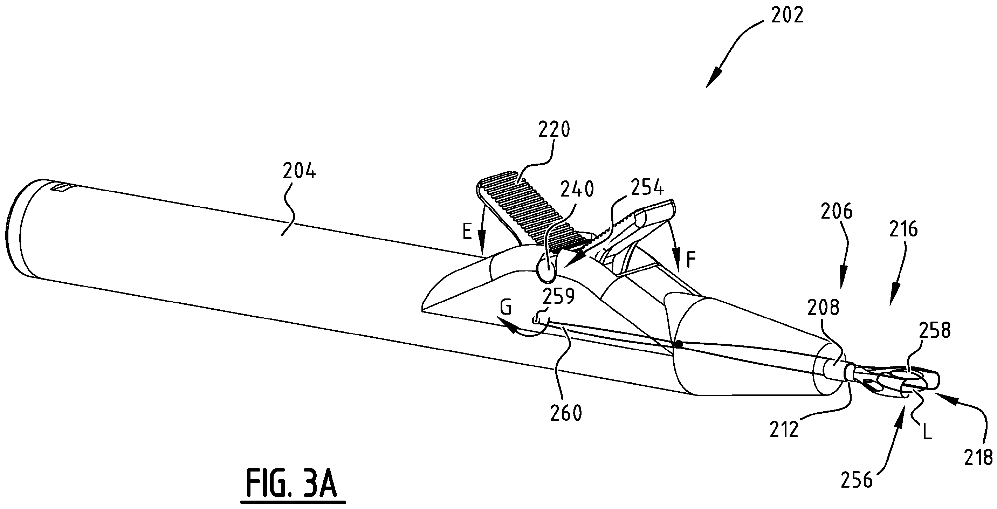

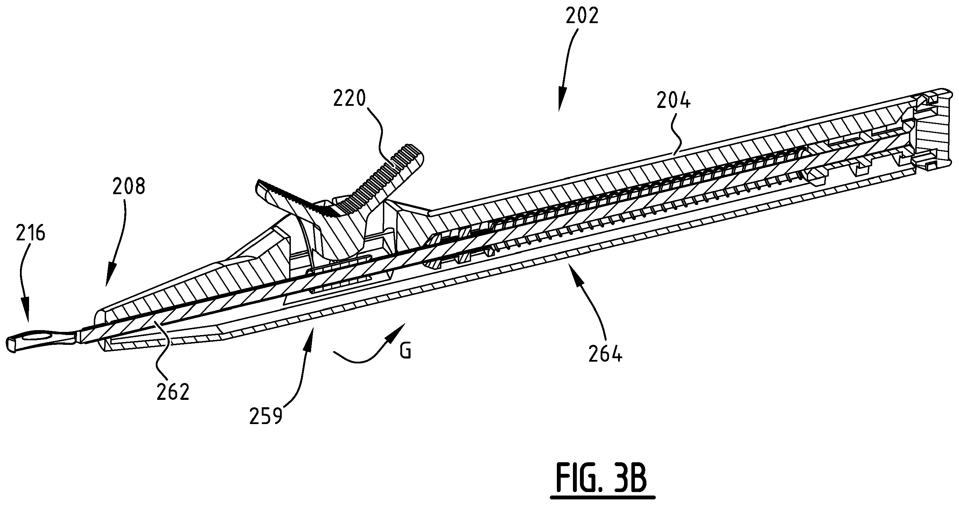

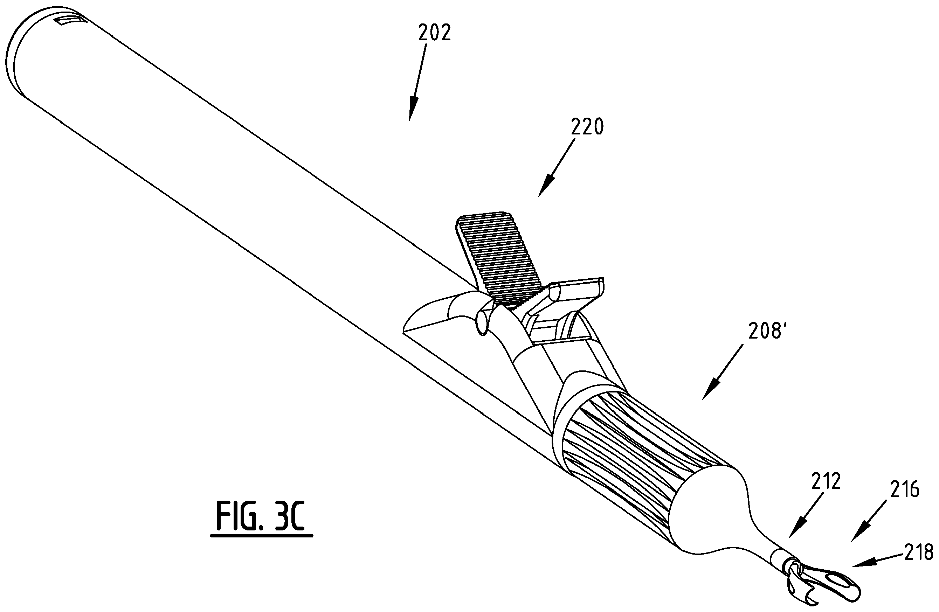

In a further alternative embodiment lens remover 202 (figures 3A-B) is provided with holder 204 in which and wherein cartridge 208 is provided movably on outer end 206. Gripper element 216 with cup-shaped grippers 218 is movable relative to cartridge 208 using operating element 220. Folding element 212 of cartridge 208 provides for the two gripper elements 218 between which lens L is placed being pressed together in use. Owing to cup shape 256 and openings 258 through which lens L protrudes partially, lens L is fixed and, when gripper elements 218 are pressed together, it is folded and partially stretched when cartridge 208 slides forward. Operating element 220 is here connected with pivot pin 240, arranged in recess 254, to holder 204. Using a rotating movement in direction G the rotation can be converted into a translation of cartridge 208 by movement in direction E or F. Said rotation is shown schematically around point 259 and the translation mechanism is shown schematically with 260. It will be apparent that diverse embodiments for such mechanisms according to the invention are possible. In the shown embodiment translation mechanism 260 (figure 3B) is arranged in holder 204. Rotation of handle 220 is converted by mechanism 259 into translation of shaft 262 of translation mechanism 260. In the shown embodiment spring mechanism 264 produces a spring force whereby gripper 216 is placed in a rest position unless handle 220 is used to move gripper 216 relative to cartridge 208 (and holder 204). The translating movement of gripper 216 is optionally combined with a rotating movement, for instance in similar manner as shown in figure 2C for lens remover 102". Lens remover 202 is optionally provided with (manually) operable cartridge 208' (figure 3C), which corresponds for the most part with cartridge 8, 8' of figures 1A,C,D.

For removal of lens L from an eye with cartridge 208, 208', lens remover 202 is placed relative to the eye extending partially through an incision. During insertion operating element 220 is pressed downward in direction E, wherein gripper elements 118 are situated largely or wholly in the interior of cartridge 208, 208'. After positioning of remover 202 element 220 is moved in direction F, whereby gripper elements 218 extend out of cartridge 208, 208'. Lens L is then positioned, optionally with an auxiliary tool, between the two gripper elements 218, wherein in the shown embodiment edges of lens L extend partially through openings 258.

After positioning of lens L, element 220 is moved in direction E by the user, whereby a rotation around hinge 258 is realized and cartridge mechanism 260 for instance ensures that cartridge 208 slides over guide elements 258, wherein lens L is folded and disappears for the most part into the interior of cartridge 208. After this, holder 204 is moved and retracted, and thereby removed from the incision, whereby lens L is removed from the eye.

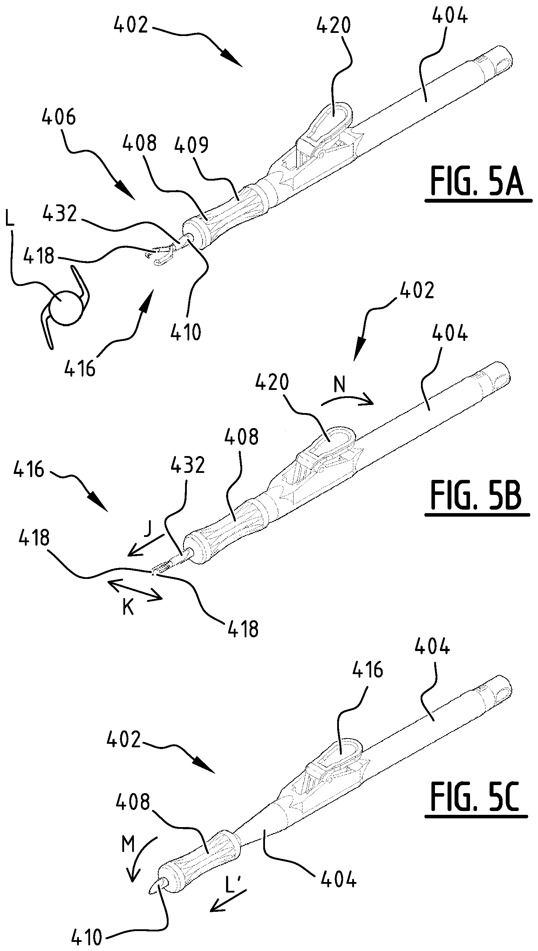

Lens remover 220 can optionally be provided with a tubular guide for the purpose of the engagement on lens L. This is for instance possible by applying guide 432, as shown in figures 5A-C. Use can optionally also be made of a spring element, such as previously shown elements 156, 264. Operating handle 220 can preferably be pressed forward in order to increase the ergonomic convenience of use.

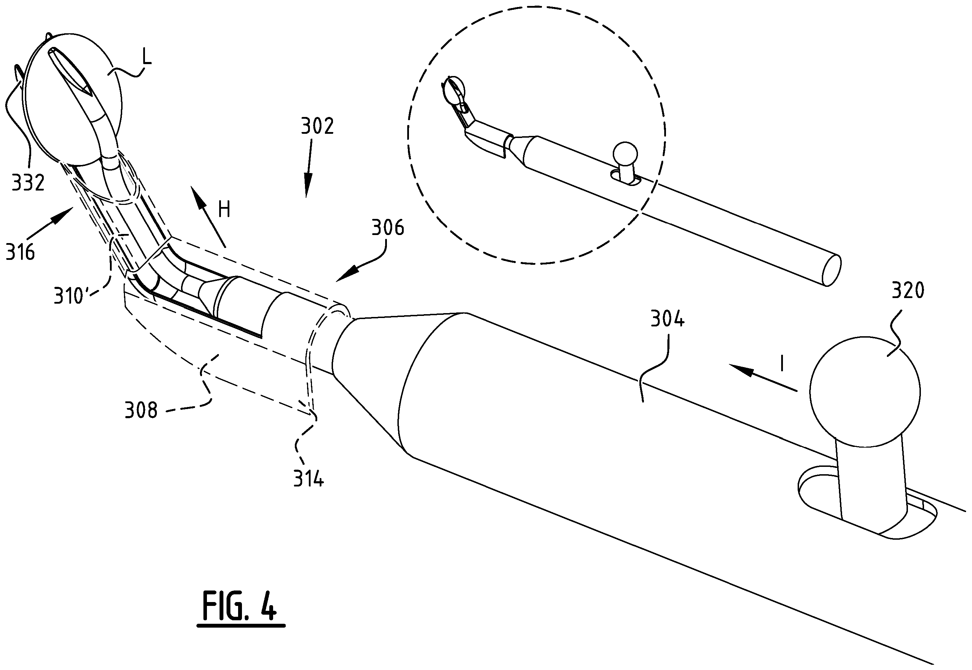

In a further alternative embodiment lens remover 302 (figure 4) is provided with holder 304 which is provided on outer end 306 with cartridge 308. Gripper 316 is provided with gripper element 318, which can move relative to guide 332 in similar manner as discussed in relation to lens remover 2 in figure 1B. After lens L being correctly positioned and preferably pre-folded, cartridge 308 is movable in direction H and here slides over gripper 118, whereby lens L is folded further and disappears for the most part into the interior of cartridge 308. The operation of gripper element 318 takes place using operating element 320.

During removal of lens L remover 302 is placed relative to an incision, wherein cartridge 308, particularly with cartridge outlet 310, extends through the incision. After remover 302 has been positioned in the eye, gripper element 318 is moved forward using the movement of operating element 320 in direction I such that lens L can be positioned relative to gripper element 318, optionally using an auxiliary tool. After this, gripper element 318 is pulled inward by operating handle 320, wherein lens L is pre-folded to some extent. Cartridge 308 is then moved in direction H over gripper element 318. This movement can take place using a mechanism or, alternatively, using a manual pushing movement by the user. This user engages here on engaging elements 314, which are preferably formed specifically for this purpose. After lens L has been introduced at least for the most part into the interior of cartridge 308, holder 304 can be retracted from the incision, whereby lens L is removed from the eye.

In an alternative embodiment for lens remover 402 (figures 5A-C) it is provided with holder 404 which is provided at outer end 406 with movable cartridge 408. In the shown embodiment cartridge 408 is provided with profiled engaging part 409. Gripper 416 is provided with cup-shaped gripper elements 418 which force a downward movement of the lens relative to the (lens) guide 432. Guide 432 is slidable in direction J to and from gripper elements 418 such that gripper elements 418 move toward each other or conversely move apart in direction K, substantially transversely of direction J. Guide 432 is slidable in direction J by means of operating element 420 in direction N. Use can be made here of a similar internal mechanism as shown in relation to other embodiments, or another suitable mechanism. After correctly positioning and engaging lens L, cartridge 408 is movable in direction L and here slides over gripper elements 418, whereby lens L is folded further and disappears for the most part into the interior of cartridge 408. In the shown embodiment cartridge 408 is rotatable in direction M around a longitudinal axis of remover 402, such that cartridge 408 is manoeuvrable over lens L.

During removal of lens L remover 402 is placed relative to an incision, wherein cartridge 408, particularly with cartridge outlet 410, extends through the incision. After remover 402 has been positioned in the eye, guide 332? is moved forward in direction J using the movement of operating element 420 in direction N, whereby gripper elements 418 move toward each other in direction K such that lens L can be positioned relative to gripper elements 418, optionally using an auxiliary tool. Lens L is here (pre-)folded. Cartridge 408 is then moved in direction L' and direction M over gripper elements 418 and lens L. This movement can take place using a mechanism or, alternatively, using a manual pushing and rotating movement by the user. This user engages here on engaging elements 409, which are preferably formed specifically for this purpose. After lens L has been introduced at least for the most part into the interior of cartridge 408, holder 404 can be retracted from the incision, whereby lens L is removed from the eye.

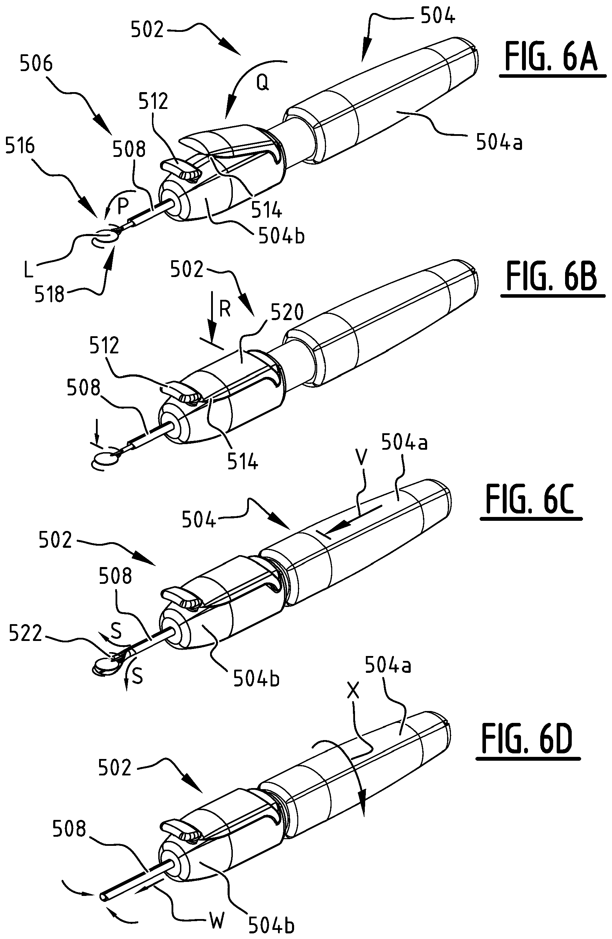

In a further alternative embodiment for lens remover 502 (figures 6A-D) it is provided with holder 504, comprising first holder part 504a and second holder part 504b, which is provided at outer end 506 with cartridge 508, optionally from a metal and/or plastic material. Holder part 504a can here take a slightly thicker form and thereby sit better in the hand of a user and provide more stability. Gripper 516 is provided with gripper elements 518, of which one element is rotatable in direction P in the shown embodiment so as to engage on lens L and clamp lens L between gripper elements 518. Clamping movement in direction P is realized using operating element 520, which can be pressed in direction Q in the shown embodiment. It is possible, if desired, to fix the operating element using positioning elements 512, 514, which can be brought into mutual engagement by pressing operating element 520 further in direction R. Arranged in cartridge 508 is film 522, for instance a so-called injection moulding film, which can be moved in forward direction and folding partially outward in direction S, optionally also using film guide mechanism 524, by movement of holder part 504b in direction V. After film 522 has at least partially surrounded lens L, cartridge 508 can be extended in direction W by rotation of holder part 504a in direction X over film 522, lens L and gripper 516. Use can be made here of a similar internal mechanism as shown in relation to other embodiments, or another suitable mechanism.

During removal of lens L remover 502 is placed in an incision, wherein cartridge 508 moves through the incision. After remover 502 has been positioned in the eye lens L is clamped by gripper elements 518, using the movement of operating element 520 in direction Q (figure 6A), by rotation of one gripper element 518 in direction P, substantially round an axis transversely of the longitudinal direction of remover 502. Lens L is here optionally (pre-)folded to some extent. Gripper element 518 is then fixed by pressing operating element 520 on in direction R (figure 6B). Film 522 is then moved outward in direction S and will surround lens L at least partially (figure 6C) by movement of holder part 504a in direction V. Cartridge 508 is then moved over film 522 and lens L in direction W. In the shown embodiment this movement is realized by rotation of holder part 504a in direction X (figure 6D). After lens L has been introduced at least for the most part into the interior of cartridge 508, holder 504 can be retracted from the incision, whereby lens L is removed from the eye.

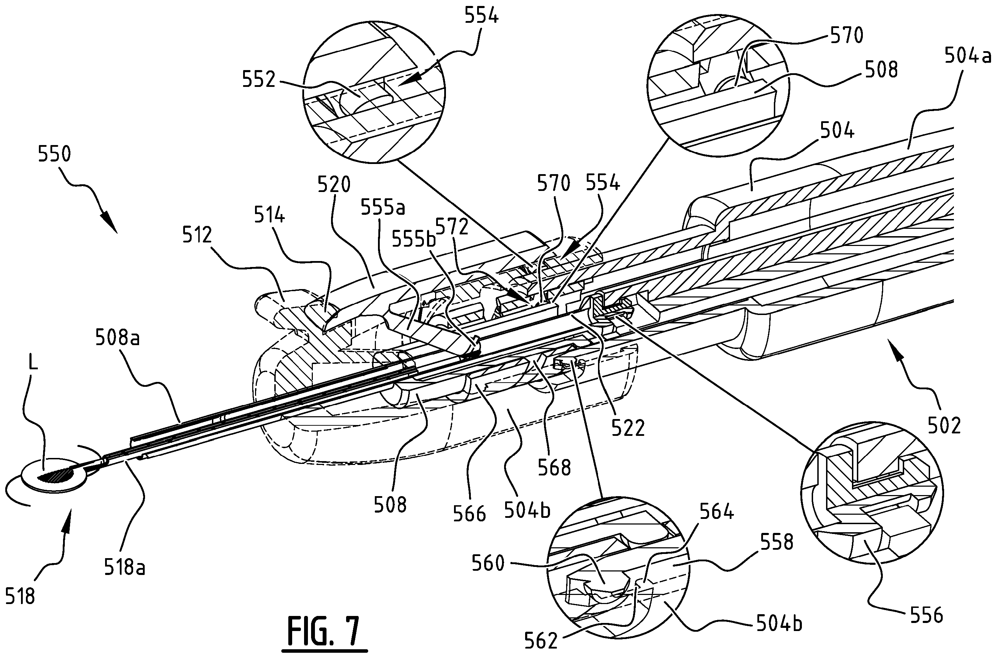

For the operation of lens remover 502 use can be made of drive mechanism 550 (figure 7). Drive mechanism 550 is activated by operating element 520, preferably without fixing it, to bring gripper elements 518 close together so that they can thereby be passed in relatively simple manner through an incision and then released. Operating element 520 is here mounted pivotally on shaft 552 and can be pressed counter to the spring action of the spring element 554. Internal part 518a of gripper element(s) 518 are moved rearward and gripper elements 518 are pressed together via catch connection 555a,b. First holder part 504a is rotatable in direction X and connected operatively via film connecting element 556 to film 522. Owing to connection 556, rotation of the first holder part 504a will result in a translation of film 522. In the shown embodiment first holder part 504a is provided with snap fingers 558 with a number of protrusions 560 which are arranged operatively in screw thread/pitch 562 of second holder part 504b. This enables a relative movement between holder parts 504 a,b. Optional edge 564 at the start of screw thread 562 of second holder part 504b prevents undesired rotation of first holder part 504a, for instance during transport. A similar edge is optionally provided at the other end of screw thread 562 in order to indicate that the outermost position of film 522 has been reached. In the shown embodiment outer end 508a of cartridge 508 and film 522 are slightly oval in order to avoid undesired rotation and to enable only translation of film 522. Transmission element 566 is provided for transmitting the (translating) rotation from first holder part 504a to cartridge 508. For this purpose element 566 is provided with recess(es) 568 for receiving protrusions 560. After receiving protrusions 560, element 566 will be co-displaced in the rotating movement of first holder part 504a. Cartridge 508 is provided with protrusions 570 which, in use, are arranged in internal screw thread 572 of element 566 and thereby convert the (translating) rotation of element 566 into a translation of cartridge 508, which can then slide with outer end 508a over lens L.

In use remover 502 is removed from the packaging. When entering through the incision made in the eye, operating element 520 is pressed without fixing it. Gripper elements 518 here have minimal dimensions and there is almost no resistance in the incision for entering the eye. As soon as gripper elements 518 have passed the incision, operating element 520 can be released and the instrument is ready to be able to grip a lens L in the eye. The edge of lens L is here positioned in the gripper elements 518. Lens L is then gripped by closing gripper elements 518 by pressing operating element 520 counter to spring action of spring element 554 and rotation of operating element 520 around shaft 552, wherein internal part 518a is moved inward and gripper elements 518 close. When operating element 520 is pressed in fully, positioning elements 512, 514 come into contact with each other for fixation. If desired, the fixation can be released by pushing further. Following fixation, film 522 is placed round lens L by sliding/moving film 522 forward by rotating first holder part 504a and thereby moving it forward, wherein film 522 slides forward and unfolds around lens L. For the stability of remover 502, and thereby the position of lens L in the eye, first holder part 504a can optionally be turned only in clockwise direction. Connection 556 converts movement of first holder part 504a into translation of film 522. A further rotation of first holder part 504a activates transmission element 566 using protrusions 570 for the purpose of transmitting the (translating) rotation of first holder part 504a to cartridge 508. Cartridge 508 moves forward with outer end 508a and will slide over film 522 with lens L therein, and compress lens L by preferably folding and stretching it. Remover 502 is then removed from the eye with lens L.

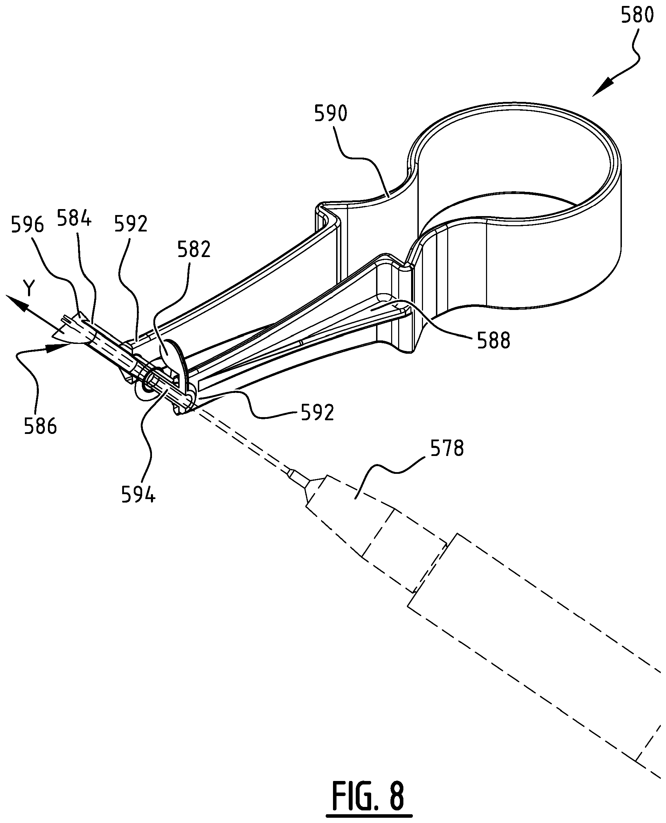

An alternative drive mechanism 580 (figure 8) provides a separate instrument 580. Instrument 580 is provided with left-right switch 582 for choosing a left eye or a right eye. Instrument 580 is further provided with guide 584 with chamfered outer end 586 for guiding remover 578 in the form of a gripper or a shown embodiment. In the shown embodiment instrument 580 is provided with strengthening ribs 588. Provided on instrument 580 are engaging positions 590 whereby outer ends 592 of instrument 580 can be moveable toward each other. Provided in the shown embodiment is cartridge 594 which moves in direction Y by movement of outer ends 592 and can thereby move over lens L, wherein lens L is preferably folded and stretched.

In use instrument 580 as drive mechanism is removed from the packaging and switch 582 is set for the left eye or the right eye. The chamfered surface 586 of the (incision) guide 584 is placed into the incision already made in the eye without instrument 580 being pressed. A user can then pass a (conventional) gripper 578 through the opening of active cartridge 594 with a second hand in order to thereby be able to grip lens L in the eye. Lens L is then gripped at the edge and preferably moved into foil 596 with the gripper(s) of remover 578. Engaging positions 590 are then pressed with the other hand, whereby outer ends 592 move toward each other. The attachment of cartridge 594 to outer end 592 moves cartridge 594 in direction Y relative to outer end 592. The forces are transmitted in controlled manner in that one or more strengthening ribs 588 are arranged. Cartridge 594 will be pushed forward through incision guide 584 and will unfold around lens L on the front side, for instance as film 596. By then moving gripper 578 with the gripped lens therein out of the eye active cartridge 594 will start folding lens L and make it small enough that it can be pulled into incision guide 584. After this, instrument 580 with the incision guide 584 inserted into the eye and lens L can be removed from the eye.

It will be apparent that drive mechanisms 550 and 580 can also be applied to other embodiments of a lens remover, both embodiments that are shown and embodiments that are not shown. It will be apparent that alternative drive mechanisms according to the invention are likewise possible.

It will be apparent that it is possible to combine diverse components and elements of the various embodiments for lens remover 2, 2', 2", 102, 102', 102", 202, 302 with each other. With an additional angular connection it is thus for instance possible to also place gripper elements 18, 118, 218 at an angle to the respective holder. It is further possible to provide operating elements 20, 20', 20", 120, 120', 122, 220, 320 integrated or conversely separately for integrated or separate movement of gripper elements 18, 118, 218, 318 and cartridge 8, 8', 108, 208, 208', 308 relative to each other and to holder 4, 104, 204, 304.

Various techniques can be used for manufacture of lens remover 2, 2', 2", 102, 102', 102", 202, 302. Casting processes can for instance be used here to form components which are then preferably assembled in a controlled environment, wherein operating elements 20, 20', 20", 122, 120', 220, 320 can then for instance be snapped into place. The various moving mechanisms and/or rotation mechanisms such as the mechanisms 142, 148 of figure 2A can be applied wholly or partially in the other shown embodiments and/or can also be applied by the skilled person in a somewhat adapted embodiment.

The present invention is by no means limited to the above described preferred embodiments thereof. The invention is defined by the claims.

## Claims

1. Intraocular lens remover (2, 2', 2", 102, 102',102", 202, 302, 402, 502) for removing an intraocular lens (IOL) (12), the lens remover comprising:
- a holder (4, 104, 204, 304, 404, 504);
- a gripper (16, 30, 116, 216, 316, 416, 516) that is movably provided in the holder and is operatively connected to the holder, and is further provided with a gripper element (18, 118, 218, 318, 418, 518);
- a cartridge (8, 108, 208, 208', 308, 408, 508) configured to move wholly or partially around or over the gripper, wherein the cartridge is connected movably to the holder; and
- a cartridge operating element (14,122) for moving the cartridge relative to the holder and the gripper, **characterized by** a gripper operating element (20, 20', 20", 120, 120', 220, 320, 420, 520) for moving the gripper element relative to the holder such that the gripper element exits the holder and engages on the lens,
such that the lens is foldable.

2. Intraocular lens remover according to claim 1, wherein the cartridge is configured to fold and/or stretch the lens.

3. Intraocular lens remover according to claim 1 or 2, further comprising a cartridge translation mechanism (166, 260) configured to translate the cartridge relative to the holder and gripper.

4. Intraocular lens remover according to claim 1, 2 or 3, further comprising a cartridge rotation mechanism (168) configured to rotate the cartridge relative to the holder and gripper.

5. Intraocular lens remover according to claims 3 and 4, further provided with a cartridge guide (170,172) which is connected operatively to the cartridge and is configured to guide the cartridge in a combined translating and rotating movement.

6. Intraocular lens remover according to any one of the foregoing claims, comprising an integrated operating element (120) in which the gripper operating element and the cartridge operating element are integrated, wherein the integrated operating element is preferably provided with a guide track (152) for movement of the cartridge and a guide (146) for movement of the gripper.

7. Intraocular lens remover according to any one of the foregoing claims, further comprising a holder rotation element (26) configured to engage on the holder for rotating the holder relative to the cartridge.

8. Intraocular lens remover according to any one of the foregoing claims, further comprising one or more lens guides (32, 32') configured to wholly or partially fold the lens with the gripper element.

9. Intraocular lens remover according to any one of the foregoing claims, wherein at least one of the one or more lens guides (432) is provided with a pressing form.

10. Intraocular lens remover according to claim 9, wherein the pressing form comprises a cup shape (218, 256, 418).

11. Intraocular lens remover according to claim 9, 10, wherein the gripper element is provided with a forming element (34, 112) for pre-folding the lens during positioning in the cartridge.

12. Intraocular lens remover according to any one of the foregoing claims, wherein the gripper element is configured to engage on the lens in a substantially upward direction.

13. Intraocular lens remover according to any one of the foregoing claims, wherein the gripper element is arranged at an angle to the holder.

14. Intraocular lens remover according to any one of the foregoing claims, further comprising a film (522) configured to surround the lens and a film guide mechanism configured to move the film relative to the gripper.

15. Method for manufacturing an intraocular lens remover according to any one of the foregoing claims, comprising the steps of forming and assembling components of the intraocular lens remover.

## Patentansprüche

1. Intraokularlinsenentferner (2, 2', 2", 102, 102',102", 202, 302, 402, 502) zum Entfernen einer Intraokularlinse (IOL) (12), der Linsenentferner umfassend:
- einen Halter (4, 104, 204, 304, 404, 504);
- einen Greifer (16, 30, 116, 216, 316, 416, 516), der in dem Halter bewegbar bereitgestellt ist und mit dem Halter wirkverbunden ist und ferner mit einem Greiferelement (18, 118, 218, 318, 418, 518) versehen ist;
- eine Kartusche (8, 108, 208, 208', 308, 408, 508), die konfiguriert ist, um sich vollständig oder teilweise um den Greifer herum oder über diesen zu bewegen, wobei die Kartusche mit dem Halter bewegbar verbunden ist; und
- ein Kartuschenbedienelement (14, 122) zum Bewegen der Kartusche relativ zu dem Halter und dem Greifer, **gekennzeichnet durch** ein Greiferbedienelement (20, 20', 20", 120, 120', 220, 320, 420, 520) zum Bewegen des Greiferelements relativ zu dem Halter derart, dass das Greiferelement aus dem Halter austritt und auf der Linse derart in Eingriff steht, dass die Linse klappbar ist.

2. Intraokularlinsenentferner nach Anspruch 1, wobei die Kartusche konfiguriert ist, um die Linse zu falten und/oder zu dehnen.

3. Intraokularlinsenentferner nach Anspruch 1 oder 2, ferner umfassend einen Kartuschenverschiebemechanismus (166, 260), der konfiguriert ist, um die Kartusche relativ zu dem Halter und Greifer zu verschieben.

4. Intraokularlinsenentferner nach Anspruch 1, 2 oder 3, ferner umfassend einen Kartuschendrehmechanismus (168), der konfiguriert ist, um die Kartusche relativ zu dem Halter und Greifer zu drehen.

5. Intraokularlinsenentferner nach den Ansprüchen 3 und 4, der ferner mit einer Kartuschenführung (170, 172) versehen ist, die mit der Kartusche wirkverbunden ist und konfiguriert ist, um die Kartusche in einer kombinierten verschiebenden und drehenden Bewegung zu führen.

6. Intraokularlinsenentferner nach einem der vorstehenden Ansprüche, umfassend ein integriertes Bedienelement (120), in dem das Greiferbedienelement und das Kartuschenbedienelement integriert sind, wobei das integrierte Bedienelement vorzugsweise mit einer Führungsbahn (152) für eine Bewegung der Kartusche und einer Führung (146) für die Bewegung des Greifers versehen ist.

7. Intraokularlinsenentferner nach einem der vorstehenden Ansprüche, ferner umfassend ein Halterdrehungselement (26), das konfiguriert ist, um auf dem Halter zum Drehen des Halters relativ zu der Kartusche in Eingriff zu stehen.

8. Intraokularlinsenentferner nach einem der vorstehenden Ansprüche, ferner umfassend eine oder mehrere Linsenführungen (32, 32'), die konfiguriert sind, um die Linse mit dem Greiferelement vollständig oder teilweise zu falten.

9. Intraokularlinsenentferner nach einem der vorstehenden Ansprüche, wobei mindestens eine der einen oder der mehreren Linsenführungen (432) mit einer Pressform versehen ist.

10. Intraokularlinsenentferner nach Anspruch 9, wobei die Pressform eine Becherform (218, 256, 418) umfasst.

11. Intraokularlinsenentferner nach Anspruch 9, 10, wobei das Greiferelement mit einem Formelement (34, 112) zum Vorfalten der Linse während eines Positionierens in der Kartusche versehen ist.

12. Intraokularlinsenentferner nach einem der vorstehenden Ansprüche, wobei das Greiferelement konfiguriert ist, um in einer im Wesentlichen nach oben gerichteten Richtung auf der Linse in Eingriff zu stehen.

13. Intraokularlinsenentferner nach einem der vorstehenden Ansprüche, wobei das Greiferelement in einem Winkel zu dem Halter angeordnet ist.

14. Intraokularlinsenentferner nach einem der vorstehenden Ansprüche, ferner umfassend eine Folie (522), die konfiguriert ist, um die Linse zu umgeben, und einen Folienführungsmechanismus, der konfiguriert ist, um die Folie relativ zu dem Greifer zu bewegen.

15. Verfahren zum Herstellen eines Intraokularlinsenentferners nach einem der vorstehenden Ansprüche, umfassend die Schritte eines Formens und Zusammenbauens von Komponenten des Intraokularlinsenentferners.

## Revendications

1. Dispositif de retrait de lentille intraoculaire (2, 2', 2", 102, 102', 102", 202, 302, 402, 502) permettant de retirer d'une lentille intraoculaire (LIO) (12), le dispositif de retrait de lentille comprenant :
- un support (4, 104, 204, 304, 404, 504) ;
- un dispositif de préhension (16, 30, 116, 216, 316, 416, 516) qui est pourvu de manière mobile dans le support et est relié de manière fonctionnelle au support, et est en outre pourvu d'un élément de préhension (18, 118, 218, 318, 418, 518) ;
- une cartouche (8, 108, 208, 208', 308, 408, 508) conçue pour se déplacer entièrement ou partiellement autour ou au-dessus du dispositif de préhension, dans lequel la cartouche est reliée de manière mobile au support ; et
- un élément d'actionnement de cartouche (14, 122) pour déplacer la cartouche par rapport au support et au dispositif de préhension, **caractérisé par** un élément d'actionnement du dispositif de préhension (20, 20', 20", 120, 120', 220, 320, 420, 520) pour déplacer l'élément de préhension par rapport au support de telle sorte que l'élément de préhension sort du support et vient en prise avec la lentille, de telle sorte que la lentille est pliable.

2. Dispositif de retrait de lentille intraoculaire selon la revendication 1, dans lequel la cartouche est conçue pour plier et/ou étirer la lentille.

3. Dispositif de retrait de lentille intraoculaire selon la revendication 1 ou 2, comprenant en outre un mécanisme de translation de cartouche (166, 260) conçu pour translater la cartouche par rapport au support et au dispositif de préhension.

4. Dispositif de retrait de lentille intraoculaire selon la revendication 1, 2 ou 3, comprenant en outre un mécanisme de rotation de cartouche (168) conçu pour faire tourner la cartouche par rapport au support et au dispositif de préhension.

5. Dispositif de retrait de lentille intraoculaire selon les revendications 3 et 4, pourvu en outre d'un guide de cartouche (170, 172) qui est relié de manière fonctionnelle à la cartouche et conçu pour guider la cartouche dans un mouvement combiné de translation et de rotation.

6. Dispositif de retrait de lentille intraoculaire selon l'une quelconque des revendications précédentes, comprenant un élément d'actionnement intégré (120) dans lequel l'élément d'actionnement de dispositif de préhension et l'élément d'actionnement de cartouche sont intégrés, dans lequel l'élément d'actionnement intégré est de préférence pourvu d'une piste de guidage (152) pour le mouvement de la cartouche et d'un guide (146) pour le mouvement du dispositif de préhension.

7. Dispositif de retrait de lentille intraoculaire selon l'une quelconque des revendications précédentes, comprenant en outre un élément de rotation de support (26) conçu pour venir en prise sur le support afin de faire tourner le support par rapport à la cartouche.

8. Dispositif de retrait de lentille intraoculaire selon l'une quelconque des revendications précédentes, comprenant en outre un ou plusieurs guides de lentille (32, 32') conçus pour plier entièrement ou partiellement la lentille avec l'élément de préhension.

9. Dispositif de retrait de lentille intraoculaire selon l'une quelconque des revendications précédentes, dans lequel au moins l'un parmi le ou les guides de lentille (432) est pourvu d'une forme de pressage.

10. Dispositif de retrait de lentille intraoculaire selon la revendication 9, dans lequel la forme de pressage comprend une forme de coupelle (218, 256, 418).

11. Dispositif de retrait de lentille intraoculaire selon la revendication 9, 10, dans lequel l'élément de préhension est pourvu d'un élément de formage (34, 112) pour préplier la lentille pendant son positionnement dans la cartouche.

12. Dispositif de retrait de lentille intraoculaire selon l'une quelconque des revendications précédentes, dans lequel l'élément de préhension est conçu pour venir en prise sur la lentille dans une direction sensiblement vers le haut.

13. Dispositif de retrait de lentille intraoculaire selon l'une quelconque des revendications précédentes, dans lequel l'élément de préhension est agencé selon un angle par rapport au support.

14. Dispositif de retrait de lentille intraoculaire selon l'une quelconque des revendications précédentes, comprenant en outre un film (522) conçu pour entourer la lentille et un mécanisme de guidage de film conçu pour déplacer le film par rapport au dispositif de préhension.

15. Procédé de fabrication d'un dispositif de retrait de lentille intraoculaire selon l'une quelconque des revendications précédentes, comprenant les étapes de formation et d'assemblage des composants du dispositif de retrait de lentille intraoculaire.
